# EUROPEAN PATENT SPECIFICATION

(11) **EP 2 226 002 B1**
(45) Date of publication and mention of the grant of the patent: **18.01.2012**
(21) Application number: 09154326.4
(22) Date of filing: 04.03.2009
(51) Int. Cl.: A61B 5/00, H04W 4/22

(54) **Improvements to body area networks**
Verbesserungen an körpernahen Netzen
Améliorations de réseaux corporels

(43) Date of publication of application: 08.09.2010
(73) Proprietor: Fujitsu Limited, Kawasaki-shi, Kanagawa 211-8588 (JP)
(72) Inventor: Abedi, Saied, Reading, Berkshire RG1 4LY (GB); Chebbo, Hind, Uxbridge, Middlesex UB8 3SY (GB)
(74) Representative: Stebbing, Timothy Charles

(56) References cited:
- WO-A-2006/111948
- US-A1- 2006 247 505
- XIAOJING YUAN ET AL: "DS-MAC: Differential service medium access control design for wireless medical information systems" 20 August 2008 (2008-08-20), ENGINEERING IN MEDICINE AND BIOLOGY SOCIETY, 2008. EMBS 2008. 30TH ANNUAL INTERNATIONAL CONFERENCE OF THE IEEE, IEEE, PISCATAWAY, NJ, USA, PAGE(S) 1801 - 1804 , XP031347062 ISBN: 978-1-4244-1814-5 * the whole document *
- MANIKANDEN BALAKRISHNAN ET AL: "Service preemptions for guaranteed emergency medium access in Wireless Sensor Networks" 16 November 2008 (2008-11-16), MILITARY COMMUNICATIONS CONFERENCE, 2008. MILCOM 2008. IEEE, IEEE, PISCATAWAY, NJ, USA, PAGE(S) 1 - 7 , XP031408166 ISBN: 978-1-4244-2676-8 * the whole document *

## Description

The present invention relates to wireless sensor networks including personal area networks and particularly, but not exclusively, to body area networks including wirelessly-communicating sensors disposed on or around, or implanted in, a human or animal body.

Various types of wireless sensor network have been proposed. Among these, the so-called Body Area Network or BAN is an example of wireless personal area networks (WPANs), used to convey information over relatively short distances.

Unlike wireless local area networks (WLANs), connections effected via WPANs involve little or no infrastructure. This feature allows small, power-efficient, inexpensive solutions to be implemented for a wide range of devices. Of particular interest is the possibility of the medical BAN (MBAN) in which sensors are used to monitor the status of a patient. A BAN employing mainly sensors for feeding sensed data to a data sink is an example of a wireless sensor network (WSN); however, more active devices, such as actuators, may be also be included in a MBAN.

Standard IEEE 802.15.4 defines the physical layer (PHY) and medium access control (MAC) sublayer specifications for low data-rate WPANs. IEEE 802.15.4 has some similarities with a standard for higher data-rate WPANs, IEEE 802.15.3. The documents IEEE Std 802.15.4-2006 and IEEE Std 802.15.3-2003.

WPANs of the type envisaged in IEEE 802.15.4 are suitable for applications such as industrial monitoring, but do not offer the kind of data reliability required for MBANs.

In medical applications, there is a requirement to reduce the costs associated with human labour while increasing the reliability and process automation and reducing human error. Sensors can provide the required intelligence, and already are widely employed in medical equipment. This includes hospital recuperative care, home care, intensive care units and advanced surgical procedures. There are many different types of sensors employed for medical applications, including external sensors for pulse, temperature etc., sensors which come in contact with body fluids, sensors used in catheters (through incision), sensors for external applications, disposable skin patches with wireless sensors, and implantable sensors.

A WPAN of sensors around a patient in a hospital or medical ward could provide multiple clinical benefits including patient mobility, monitoring flexibility, extension of monitoring into care areas that are currently unmonitored, reduced clinical errors and reduced overall monitoring costs. Body worn sensors may include various sensor types on a single patient body. They require a capability to be applied or removed quickly from the patient's body.

On an individual basis, such sensors may have bit rates of as low as 1-2 kbps per patient and on an aggregate basis they may require a 10 kbps bit rate. A range of as little as a few metres may be adequate. However, medical WSN applications are mission critical applications in the clinical environment. Robust wireless links for bounded data loss and bounded latency, capacity for patient and sensor density, coexistence with other radios, battery life for days of continuous operations and small form factors for body worn devices, are among the requirements for medical WSNs or MBANs. These requirements can be satisfied through utilization of techniques such as diversity and error control techniques in the time and frequency domain, including Forward Error Correction (FEC) and Adaptive Repeat reQuest (ARQ), low duty cycle TDMA for sensor information rate, and more efficient small antennas.

Efforts are therefore in progress to define a further standard IEEE 802.15.6 which aims to define the properties of Body Area Networks, particularly for medical applications. One of the key requirements of IEEE 802.15.6 is high reliability for medical applications. This is even more important for emergency situations where the life of the patient depends on the reliability of wireless links in medical WSN applications. Existing standards such as IEEE 802.15.4 have been designed for commercial application with no consideration of such emergency life saving scenarios.

In particular, there is a need to address the issue of ensuring reliability of communications with network devices such as sensors involved in such an emergency situation.

WO 2006/111948A discloses a network and a method according to the preamble of each independent claim. A personal activity monitoring apparatus is operable to provide, to a remote server, decision data such as "occurrence of the emergency" by comparing at least one body activity with a threshold.

Xiaojing Yuan et al, "DS-MAC: Differential service medium access control design for wireless medical information systems", 20th August 2008, XP031347062 ISBN:978-1-4244-1814-5, discloses a medical information system with a MAC protocol based on IEEE 802.11e to provide differentiated service between normal and emergency/warning medical information. There are three kinds of frame defined in the protocol, each corresponding to one of three classes of health monitoring traffic: highest priority emergency data, high priority warning data, and lower priority routine data.

Manikanden Balakrishnan et al.: "Service preemptions for guaranteed emergency medium access in Wireless Sensor Networks", 16 November 2008, XP031408166 ISBN: 978-1-4244-2676-8 discloses use of the QoS framework of the IEEE 802.11e EDCA standard to define emergency frames which allow emergency traffic in a WSN to pre-empt normal traffic.

### Summary of the Invention

According to a first aspect of the present invention, there is provided a wireless sensor network of devices including one or more sensors for monitoring an entity, including:
a said sensor arranged to detect a value of a parameter related to the entity and to wirelessly transmit information to another device in the network;
a coordinator arranged to receive said wirelessly transmitted information; and
determining means responsive to said detected value or said transmitted information to determine existence of an emergency condition of the entity, the determining means arranged to transmit a declaration of the emergency condition to at least one other device in the wireless sensor network; characterised in that
information is transmitted in the network via MAC frames, and the determining means is arranged to make the declaration of the emergency condition by setting a MAC frame identifier to an emergency notification frame type.

The entity is, for example, a living body such as a human being monitored for medical purposes.

In the above network the determining means may be located in any of the sensor, the coordinator, or, when the network is connected to a central monitor in communication with the coordinator, may be provided by the central monitor.

Whatever its location, the determining means may make the determination of the emergency condition (or more precisely, the coming into existence of the emergency condition) by comparing the detected value or the transmitted information with at least one threshold value. For example, two thresholds may be used to define a range.

As already stated, the determining means is operable to determine non-existence of the emergency condition (or in other words a lifting of the emergency condition) by comparing the detected value or the transmitted information with at least one threshold value.

As already mentioned, the determining means is arranged to transmit a declaration of the emergency condition or lifting the emergency condition to at least one other device in the wireless sensor network. This other device, preferably, is responsive to the declaration to send an acknowledgement to the determining means. In the case where the determining means is arranged to determine the non-emergency condition, preferably it is operable to declare this as well to the other device(s).

The network can include a IEEE 802.15.6-based MBAN. In a preferred application, the above-mentioned entity is a living body, the sensor is for sensing a life parameter of the living body of a patient, and the emergency condition is a medical emergency.

According to a second aspect of the present invention, there is provided a sensor for use in the wireless sensor network and including the determining means.

According to a third aspect of the present invention, there is provided a coordinator for use in the wireless sensor network and including the determining means.

According to a fourth aspect of the present invention, there is provided a method of declaring an emergency condition in a wireless sensor network, the wireless sensor network comprising a plurality of devices including sensors arranged for monitoring at least one entity, the method comprising steps of:
sensing a value of parameter related to the entity by one of the sensors;
wirelessly transmitting information by said sensor to another device in the network;
determining existence or non-existence of an emergency condition affecting said entity by using said sensed value or said transmitted information; and
declaring existence or non-existence of the emergency condition to at least one other device in the network; characterised in that
information is transmitted in the network via MAC frames, and said declaring step is performed by setting a MAC frame identifier to an emergency notification frame type.

According to a further aspect of the present invention, there is provided a frame format for use in a network of devices performing data transfers by wireless communication, the frame format defining a frame control field which includes a subfield for denoting an emergency frame used to declare existence of an emergency condition to other devices in the network. Such a frame format may be used within a superframe structure as proposed in IEEE 802.15.4, for example.

Further aspects of the present invention provide software which, when executed by a processor of a sensor device or a coordinator of a wireless sensor network, provides the above sensor or coordinator respectively. Such software may be stored on a computer-readable medium.

### Brief Description of the Drawings

For a better understanding of the present invention, and to show more clearly how it may be carried into effect, reference will now be made, by way of example only, to the following drawings in which:
Figure 1 illustrates protocol layers in an IEEE 802.15.4 WPAN;
Figure 2 illustrates possible PHY bands of the IEEE 802.15.4 WPAN;
Figure 3 illustrates Star and Peer-to-Peer topologies of a WPAN;
Figure 4 shows the structure of a superframe in a beacon-enabled IEEE 802.15.4 WPAN;
Figures 5 to 8 illustrate possible modes of data transfer between a network device and a co-ordinator in a IEEE 802.15.4 WPAN;
Figure 9 shows a frame format used for a data frame in a IEEE 802.15.4 WPA
Figure 10A shows the structure of a Frame Control field in the frame format of Figure 9;
Figure 10B is a table of hitherto-defined values of frame type bits in the Frame Control field of Figure 10A;
Figure 11A shows part of the frame format used for a MAC command frame in IEEE 802.15.4;
Figure 11B is a table of hitherto-defined values of a command frame identifier in the frame format of Figure 11A;
Figure 12 is a flowchart of steps in a first procedure for declaring an emergency in an embodiment of the present invention;
Figure 13 is a flowchart of steps in a first procedure for lifting an emergency in an embodiment of the present invention;
Figure 14 is a flowchart of steps in a second procedure for declaring an emergency in an embodiment of the present invention;
Figure 15 is a flowchart of steps in a second procedure for lifting an emergency in an embodiment of the present invention;
Figure 16 is a flowchart of steps in a third procedure for declaring an emergency in an embodiment of the present invention;
Figure 17 is a flowchart of steps in a third procedure for lifting an emergency in an embodiment of the present invention;
Figure 18 shows the novel structure of the Frame Control field proposed in an embodiment of the present invention;
Figure 19 is a table of possible values of frame type bits in the Frame Control field of Figure 18;
Figure 20 shows the structure of a Frame Control field in a frame format modified in accordance with another embodiment of the present invention;
Figure 21 is a table of frame type values in the Frame Control field of Figure 20; and
Figures 22A and B show a modification of the command frame identifier of Figures 11A and B in another embodiment of the present invention.

Before explaining the embodiments of the present invention, some background explanation will be given of those parts of IEEE 802.15.4 which are expected to have relevance for the IEEE 802.15.6 standard currently under development, and/or for Body Area Networks including MBANs.

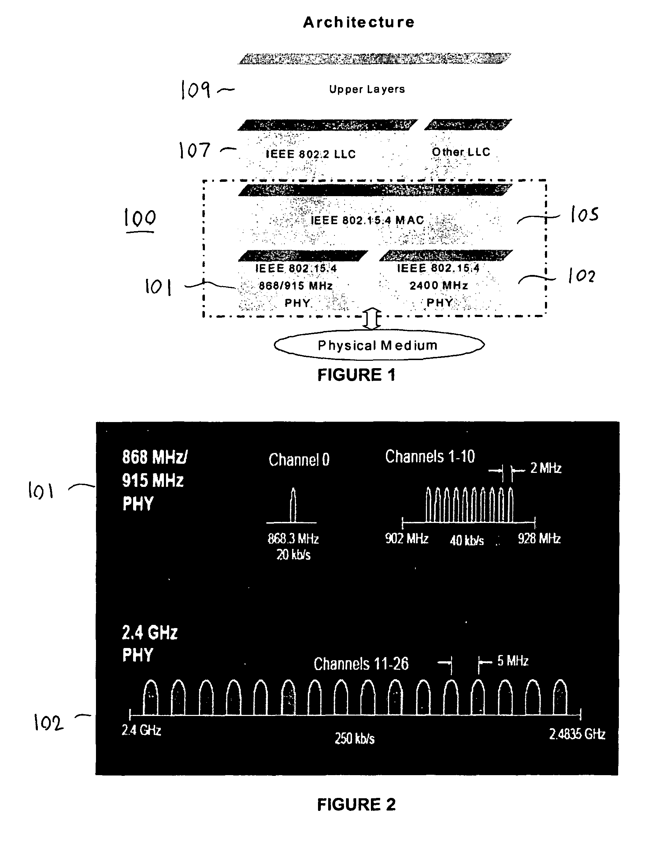

Figure 1 shows the general architecture of a IEEE 802.15.4 WPAN, labelled 100, in terms of the layered OSI model, in which the physical medium is accessed via a PHY layer containing the radio transceiver and its low-level control. As shown, there are two alternative frequency bands 101, 102 for the PHY, which are illustrated in Figure 2. The lower frequency band 101 provides a single 20kb/s channel centred on 868.3MHz, and/or ten channels each of 40kb/s centred on 915MHz. The higher frequency band 102 provides 16 channels each of 250kb/s and centred on a frequency of 2.44 GHz. Which of these bands is used will depend on local regulatory requirements.

Access to the PHY is provided by a MAC (Medium Access Control) sublayer indicated by 105 in Figure 1. Above this, and external to the WPAN 100 as such, are provided a LLC (Link Layer Control) allowing access to the WPAN from other networks; this may be in accordance with the IEEE 802.2 standard, or of another type. Finally, upper layers 109 above the LLC include a network layer to provide network configuration, manipulation, and message routing, and an application layer which provides the intended overall function.

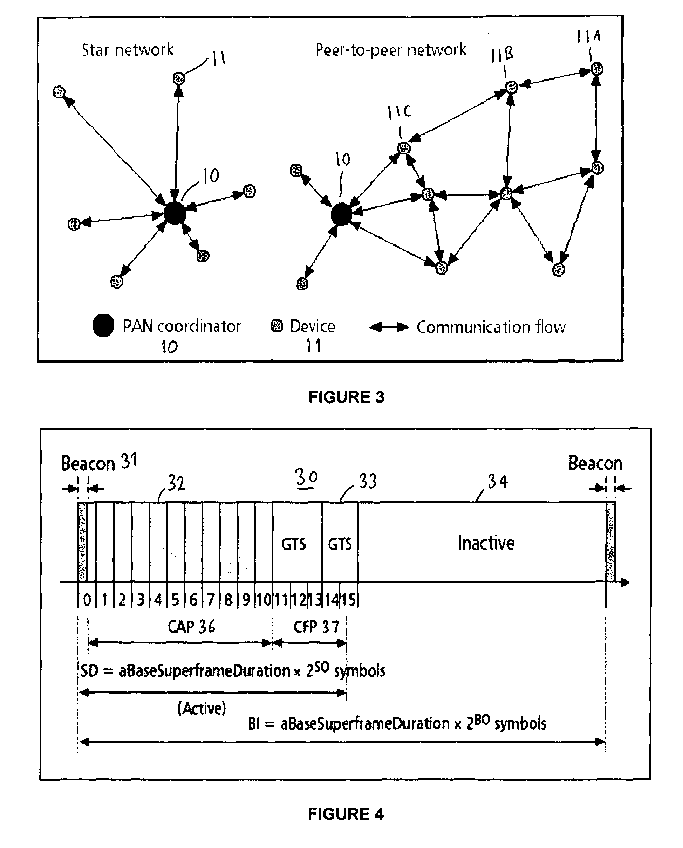

One task of the MAC sublayer is to control the network topology. Star and peer-to-peer are two known topologies in communications networks, and both are provided for in IEEE 802.15.4. In both cases, the topology distinguishes between two basic kinds of network node: devices and coordinators. As shown in Fig.3, in the Star topology a number of devices 11 communicate directly with a central co-ordinator 10; whilst in the peer-to-peer configuration, communications by a device 11A with the communicator are made along one or more hops with intermediate devices 11 B and 11C acting as relays. The coordinator acts as the access point to the upper layers; in the case of a WSN, it acts as the sink for the data collected by the sensors. Given that the communication range of each device may be very limited (a few metres), the peer-to-peer topology allows a greater area to be covered. The topology may be dynamic, changing as devices are added or leave the network.

In the case of MBANs, for example, a star network would be appropriate in the situation where a coordinator is provided at each patient site (such as a hospital bed), exchanging signals with devices on a single patient. Peer-to-peer would be a more appropriate topology where one coordinator was provided to serve a number of patients (the coordinator might be located at a fixed point in a hospital ward). Thus, whilst the devices 11 will generally be mobile the coordinator may be either mobile or fixed. Peer-to-peer networks may also be more suited to fast-changing environments where it is required to set up or change the network quickly, or to allow self-organisation and self-healing of the network. Self-healing may include, for example, establishing a new coordinator in the event that an existing coordinator has failed or left the network.

Multiple star and/or peer-to-peer networks may be set up in the same location such as a hospital, each with their own coordinator. In this case it will be necessary for the respective coordinators to collaborate in order to avoid mutual interference and to allow sharing or collation of data. In IEEE 802.15.4 such networks are called clusters, and provision is made for establishing an overall coordinator for the clusters as well as for dividing and merging clusters.

Nodes in a WPAN may be constituted by units of varying capabilities. Generally, the role of coordinator will require a relatively capable apparatus with some processing power and transceiver capable of handling transmissions from multiple sources simultaneously. This in turn will necessitate a sufficient provision of electrical power (in some cases, it may be mains powered). On the other hand, other devices in the network may have more limited processing ability and access only to battery power, and may even be so simple as to be unable to act as a relay hop. Devices with very low power availability may be shut down most of the time and only "wake up" occasionally, for example to transmit sensor data to another node. Thus, the IEEE 802.15.4 standard distinguishes between "full-function" and "reduced function" devices. Availability of power is a particular issue for MBANs in which sensors may be implanted within a body and thus unable to have a large or rechargeable battery.

Two types of WPAN envisaged in IEEE 802.15.4 are beacon-enabled and non beacon-enabled.

In a beacon enabled network, the coordinator transmits a beacon periodically and devices listen periodically to that beacon to synchronize to the network and to access the channel. The channel access is in units of "frames" transmitted sequentially within a "superframe" according to a superframe structure as shown in Figure 4, which is defined by the coordinator. Each superframe 30 consists of two parts: active and inactive. The active part is divided into a contention access period CAP 36, followed by an optional contention free period CFP 37 for guaranteed access for applications with quality of service requirement.

As indicated by the vertical divisions in Figure 4, the superframe is divided into 16 equally-spaced time slots each capable of carrying a frame of data from the coordinator or from a device. Thus, considering the devices associated with one coordinator, only one device may be in communication with the coordinator at a time during each successive time slot within the superframe. First comes a slot 31 for a beacon frame (see below) transmitted by the coordinator. After this, several slots 32 are provided within the CAP, allowing data transmission to or from devices on a contended basis, following the known CSMA-CA algorithm. Briefly, in CSMA-CA, each time a device wishes to transmit within the CAP, it waits for a random period. If the channel is found to be idle, following the random backoff, the device transmits its data. If the channel is found to be busy following the random backoff, the device waits for another random period before trying to access the channel again.

Next there follow one or more guaranteed time slots GTS 33 of the CFP, and as shown, each of these may extend over more than one basic time slot. As implied by the term CFP these GTSs are not contended for by the network devices, but rather each is reserved by the coordinator for exclusive use by a network device on a TDMA basis. Allocation of the GTSs may change, however, from one superframe to the next. After the expiry of the inactive period, the next superframe is marked by the coordinator sending another beacon frame 31. Devices can go to sleep during the inactive period 34 of the superframe. Thus, by extending the length of the inactive period 34, battery power of devices can be conserved as much as possible.

In the non beacon enabled network, the coordinator is not required to transmit a beacon for synchronization unless it is requested to do so (e.g. for network discovery purposes). The channel access is not restricted by the superframe structure and devices are asynchronous, performing all data transfers by CSMA-CA. They can follow their own sleeping pattern according to a certain protocol such as sensor - MAC (WiseMAC) in which devices having no data to send can remain idle ("asleep") for most of the time, and the coordinator prefaces each data frame by a wake-up preamble, to ensure that the receiving device is awake when the data arrives.

For an MBAN application, the coordinator is external to the body or bodies being monitored. It may be a PDA, a mobile phone, a bedside monitor station or even a sufficiently-capable sensor which on a temporary basis acts as a coordinator. As mentioned above, the coordinator in the beacon enabled network is in charge of providing synchronization and channel access to network devices. The start and end of a superframe is also defined by the coordinator. The coordinator has two main features of potential communications to other networks and access to a sufficient power supply, for example by easy replacement of the charged batteries.

A central care and monitoring unit (below, "central monitor") may also be provided for overall supervision of a network possibly containing several coordinators. This may take the form of a room with monitoring equipments capable of receiving continuous or occasional streams of emergency data from multiple patients. There will typically be nurses or medical specialists stationed in the central unit who are continuously watching and monitoring the patients' data. They will take actions in response to change in patients' conditions. The central care and monitoring unit may be connected wirelessly to the or each coordinator (in which case it may be considered part of the MBAN) or it may have a wired connection to each coordinator (in which case it may be considered as outside the MBAN as such)

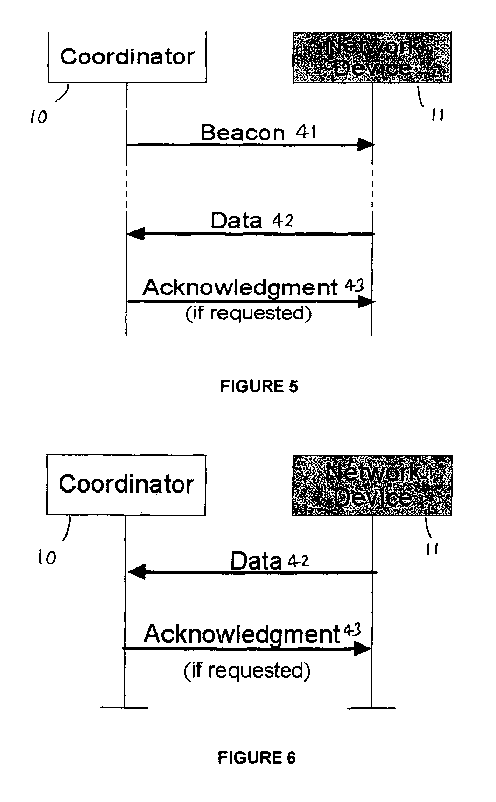

Figures 5 to 8 illustrate data transfers between a device and a coordinator in a IEEE 802.15.4 network. Three basic types of transfer are defined in IEEE 802.15.4:
(i) data transfer to a coordinator as recipient to which a device (sender) transmits its data - used in both star and peer-to-peer topologies;
(ii) data transfer from a coordinator as sender in which the device receives the data - used in both star and peer-to-peer topologies; and
(iii) data transfer between two peers - used in peer-to-peer networks only.

Figures 5 and 6 depict a transfer from the device (Network Device 11) and coordinator (Coordinator 10) for both the beacon-enabled and non beacon-enabled case respectively. The difference is that in the beacon-enabled case the device 1 must wait to receive a beacon frame 41 from the coordinator prior to sending the data (data frame 42) using CSMA-CA in the CFP, or using a GTS in the CAP; whilst in the non beacon-enabled case there is normally no beacon frame and the device 11 sends a data frame 42 at will using CSMA-CA. In either case, the coordinator acknowledges the successful reception of the data by transmitting an optional acknowledgment frame or ACK 43. These different types of frame are explained in more detail below.

If the recipient is unable to handle the received data frame for any reason, the message is not acknowledged. If the sender does not receive an acknowledgment after some period, it assumes that the transmission was unsuccessful and retries the frame transmission. If an acknowledgment is still not received after several retries, the sender can choose either to terminate the transaction or to try again. When the acknowledgment is not required, the sender assumes the transmission was successful.

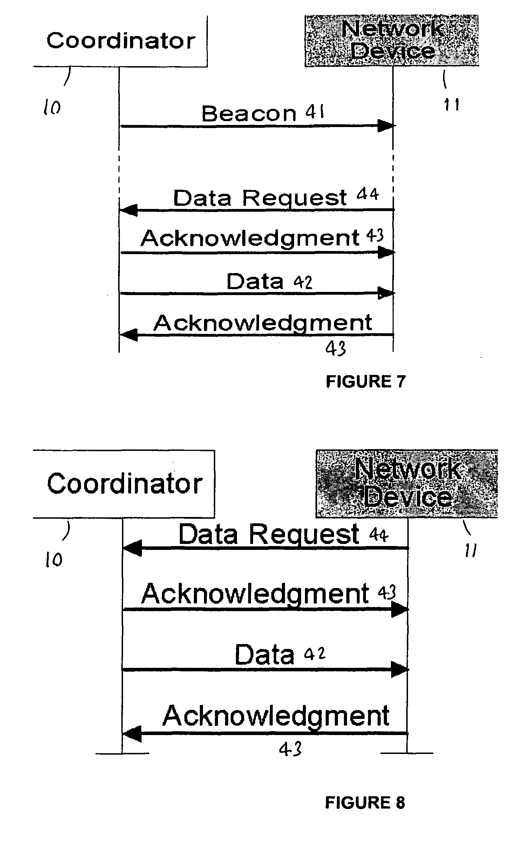

Figures 7 and 8 illustrate data transfer from a coordinator 10 to a device 11. When the coordinator wishes to transfer data to a device in a beacon-enabled WPAN (Figure 7), it indicates in the beacon frame 41 that the data message is pending. The device periodically listens to the beacon frame and, if a message is pending, transmits a data request (MAC command) 44 requesting the data by CSMA-CA. The coordinator 10 acknowledges the successful reception of the data request by transmitting an acknowledgment frame 43. The pending data frame 42 is then sent using slotted CSMA-CA or, if possible, immediately after the acknowledgment. The device 11 may acknowledge the successful reception of the data by transmitting an optional acknowledgment frame 43. The transaction is now complete. Upon successful completion of the data transaction, the message is removed from the list of pending messages in the beacon.

In the non beacon-enabled case, the coordinator 10 which has data ready for a particular device 11 has to wait for a data request 44 from the device concerned, sent on a contention basis. Upon receiving such a request, the coordinator sends an acknowledgement frame 43 (this can also be used to signify that no data is ready, if that is the case), followed by the data frame 42, in response to which the device 11 may send another acknowledgement frame 43 in return.

For simplicity, the above procedures have considered only the above cases (i) and (ii) of data transfers between the device and coordinator, but in a peer-to-peer network, as already mentioned, data transfers will generally take place via mechanism (iii), involving one or more intermediate nodes, which increases the risk of collision and the delays involved.

As indicated in Figures 5 to 8, communications in a IEEE 802.15.4 network involve frames of four different types:
- beacon frame 41, used by a coordinator to transmit beacons
- data frame 42, used for all transfers of data
- acknowledgment frame 43, used for confirming successful frame reception
- MAC command frame 44, used for handling all MAC peer entity control transfers such as data requests.

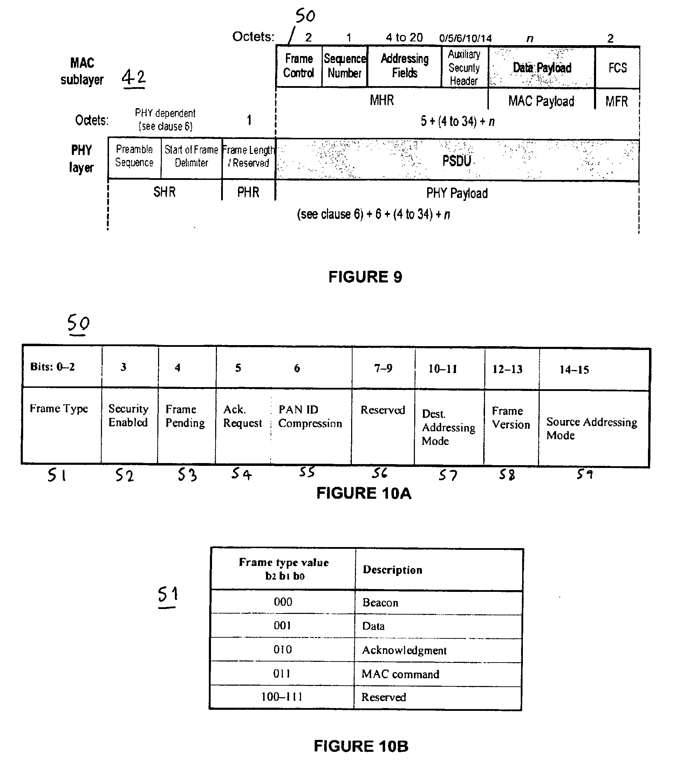

The structure of each of the four frame types is quite similar, and is shown in Figure 9 for a data frame 42 by way of example. In the Figure, the two horizontal bars represent the MAC sublayer and the PHY layer respectively. Time progresses from left to right, and the time length of each successive field of the frame is shown (in octets) above the field concerned. Every frame consists of a sequence of fields in a specific order, these being depicted in the order in which they are transmitted by the PHY, from left to right, where the leftmost bit is transmitted first in time. Bits within each field are numbered from 0 (leftmost and least significant) to k - 1 (rightmost and most significant), where the length of the field is k bits.

The data to be sent via the data frame 42 originates from the upper layers. The data payload is passed to the MAC sublayer and is referred to as the MAC service data unit (MSDU). The MAC payload is prefixed with an MAC Header MHR and appended with a MAC Footer MFR. The MHR contains the Frame Control field 50 (see below), data sequence number (DSN), addressing fields, and optional auxiliary security header. The MFR is composed of a 16-bit frame check sequence FCS. The MHR, MAC payload, and MFR together form the MAC data frame, (i.e., MPDU). The MPDU is passed to the PHY as the PHY service data unit PSDU, which becomes the PHY payload. The PHY payload is prefixed with a synchronisation header SHR, containing a Preamble Sequence and a start-of-frame delimiter SFD, and a PHY header PHR containing the length of the PHY payload in octets. The preamble sequence and the data SFD enable the receiver to achieve symbol synchronization. The SHR, PHR, and PHY payload together form the PHY packet (the PHY protocol data unit PPDU).

The beacon frame 41, acknowledgement frame 43 and MAC command frame 44 have a similar structure, except that the MAC payload has a different function in each case, the acknowledgement frame having no MAC payload. Also, the beacon frame 41, the acknowledgement frame 43 and MAC command frame 44 originate in the MAC sublayer without involvement of the upper layers.

The frame control field 50 used in each type of frame is shown in more detail in Figure 10A. It consists of 16 bits assigned to subfields for different purposes as illustrated. In particular, the first three bits of the field denote the Frame Type 51: beacon frame 41, data frame 42, acknowledgement frame 43, or MAC command frame 44. The way the frame type is signified is shown in Figure 10B. Following the frame type bits 51 is a single-bit Security Enabled subfield 52 denoting whether or not security is enabled by the MAC sublayer. This is followed by a Frame Pending subfield 53 to indicate whether the sender has more data for the recipient. Next is an Ack. Request subfield 54 to indicate whether an acknowledgement is requested from the recipient. After this follow some further sub-fields 55, to 59 which are used for addressing purposes or reserved in the current IEEE 802.15.4 specification.

As mentioned, Figure 10B is a table of the possible bit values for the Frame Type subfield 51, showing that values 100 and 101 are unused in the IEEE 802.15.4 specification.

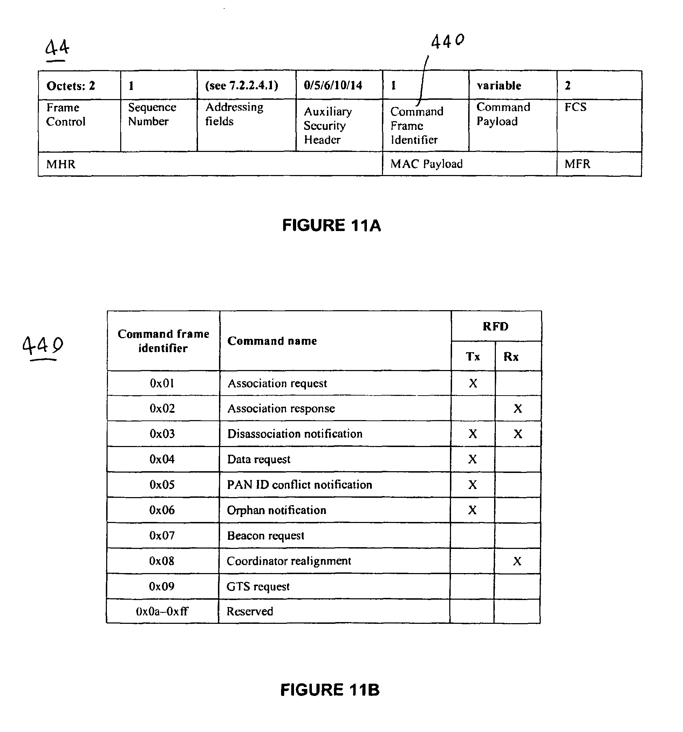

The MAC command frame 44 is quite similar in structure as shown in Figure 11A. In this case the payload includes a command frame identifier 440 to identify the type of command represented by the MAC command frame. Various types of command are defined in IEEE 802.15.4 as shown in the table of Figure 11B, showing possible values of identifier 440 with some values reserved for future use.

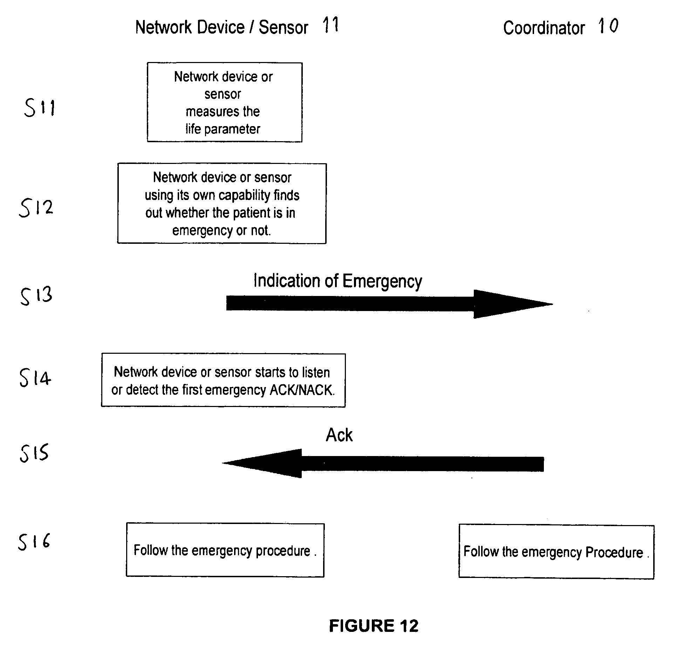

Having outlined the background of the present invention, some embodiments will now be described with reference to Figures 12 to 21.

The present invention addresses, for example, the situation in which at least one patient is being monitored via a MBAN of sensors disposed on or around, or implanted in, the patient's body. (Depending on the context, the terms "MBAN" and "network" may be applied either to a plurality of sensors with their coordinator provided for a single patient, or collectively to all the sensors/coordinator(s) of multiple patients). It is assumed that at least some of the sensors are involved in sensing one or more parameters, such as heart rate, which might indicate a life-threatening situation for the patient. Embodiments of the present invention provide methods for declaring an emergency state, and for lifting the emergency state, by providing a protocol which includes emergency acknowledge (i.e. ACK/) mechanisms to support the patient during such life threatening situations. Three different protocols or trigger types are proposed depending on the capabilities of network devices to process or analyze the sensed data or measurements.

In the following Figures, "in emergency" refers to the patient being in a life-threatening situation owing to some sensed parameter(s) having reached a critical level; "ACK" corresponds to the above-described acknowledgement frame 43, and "emergency procedure" refers to any steps that may be taken in response to an emergency being declared. An "emergency operating state" is some manner of operating a network device in emergency, whilst a "normal operating state" is a condition which applies when a network device is not in emergency.

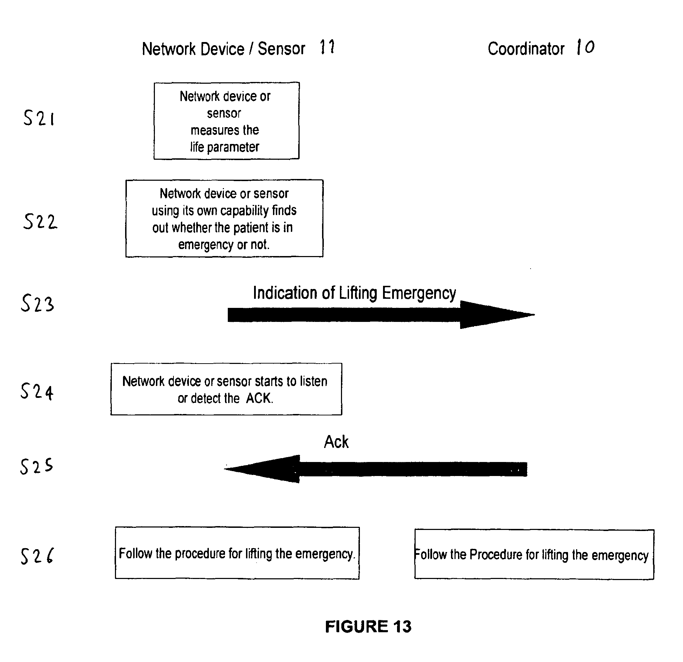

In a first protocol, flowcharts of which are shown in Figures 12 and 13, the sensor (network device) itself decides whether or not to declare an emergency state, and to lift the emergency state. First, the process for declaring the emergency is described with reference to Figure 12. It is assumed that the sensor starts from a "normal" (non emergency) state. This will typically involve the sensor measuring a certain parameter relating to the patient (henceforth called "life parameter") at a certain time interval, which could be set internally by the sensor or instructed from outside, by or through the coordinator. For simplicity, the case of one sensor "in emergency" will be considered, but the protocol to be described may apply to a group of sensors (such as all sensors monitoring the same patient).

Declaring the State of Emergency, Trigger Type 1 (Figure 12):
S11: At the predetermined time interval, the network device or sensor 11 measures the life parameter. The sensor may transmit the sensed value wirelessly to the coordinator (either directly, in as Star topology, or via one or more hops in the case of a Peer-to-Peer network). However, it is not essential for every (or any) sensed value to be transmitted in the non-emergency state. In any case, the sensed value will be stored at least temporarily in the sensor.
S12: The network device or sensor using its own capability finds out whether the patient is in emergency or not. More precisely, it is determined whether the status has changed from non-emergency to emergency. For example, it checks whether the patient's heart rate has exceeded a threshold rate. Thus, normally, the sensor will have some form of memory for storing the threshold. Depending on the life parameter involved, more than one threshold level might be used to set a range. For example, a blood pressure sensor could determine an emergency any time the sensed value falls outside an acceptable range bounded by upper and lower thresholds of, say 160/120 and 80/60 (systolic/diastolic pressure in mm Hg).
S13: The network device or sensor sends an indicator of emergency status to the coordinator 10. The way in which this is signified is explained below. For now, it is noted that the indicator is sent as a special kind of frame which, depending on the network configuration, may be sent either through direct transmission or via other devices used as relays. In the latter case a destination ID contained in the indicator can be used to show the intended destination.
S14: The network device or sensor 11 starts to listen for or detect an acknowledgement (ACK) from the coordinator 10. It is important that the sensor not only send the declaration of emergency status, but also ensures that it has been received so that appropriate action can be taken by the coordinator or higher authority.
S15: The coordinator 10 sends back the ACK to the network device 11. In a frame-based system such as IEEE 802.15.4, the acknowledgement frame is normally used for this purpose, and it is expected that this frame type will also be defined in IEEE 802.15.6.
S16: Some emergency actions will follow. For example, the sensor 11 may increase the frequency of measurement of the life parameter, and/or frequency of sending messages to the coordinator 10; this could include beginning to send its sensed readings to the coordinator even if the sensor was not doing this before. The coordinator in turn allows for the increased frequency, for example by providing a GTS for the sensor concerned. In other words the coordinator may begin to provide a predetermined quality of service to the sensor. The coordinator may take additional action such as alerting a central monitoring station (not shown), or (in the case where the same network is monitoring a plurality of patients) divert resources to other sensors on the same patient, allowing closer scrutiny of that patient's other life parameters. This could include the coordinator notifying all other sensors in the MBAN, or a group of related sensors relevant to the life parameter of concern, of the emergency status. Other emergency actions would include the coordinator sending some form of alert outside the system such as an audible alarm or pager message.

Note that the emergency actions are likely both to increase the power consumption of the sensor concerned, and to cause bandwidth allocated by the coordinator to be diverted from other sensors; thus, the emergency state should not be prolonged indefinitely (see below).

Note also that in the above protocol, it may not always be necessary for the sensor to transmit the sensed value: it may be sufficient for the sensor to transmit information (such as the sensed value, or perhaps only indicator of emergency status) only in the event that a threshold has been reached. Instead, or in addition, the sensor may save up a number of sensor readings for transmission all at once. In these ways, possibly unnecessary power consumption and transmissions of routine sensor data in the network can be reduced to a minimum.

Suppose that the sensed life parameter returns to a non-critical level; the sensor can use this fact to lift the emergency status, allowing the coordinator to give greater priority to other sensors and tasks. The procedure for this is shown in Figure 13. It is assumed that the sensor concerned is presently in an emergency condition, including, for example, sensing the life parameter at a relatively short time interval and sending frequent messages to the coordinator

Declaring lifting of Emergency, Trigger Type 1 (Figure 13):
S21: Network device or sensor 11 measures the life parameter. The sensed value will normally be transmitted directly to the network as already noted; this may be important in the emergency state for allowing the coordinator and any outside entity to monitor the patient's emergency situation.
S22: The network device or sensor using its own capability finds out whether the patient is still in emergency or not. More precisely, it is determined whether the state has changed from emergency to non-emergency (lifting of emergency). For example, if the sensed parameter falls below a threshold level, or within an acceptable range, it may be assumed (at least as far as that sensed parameter is concerned) that the patient is no longer in a critical state. By way of illustration, in the case of a pulse sensor, a pulse value within the range of say 50-120 (beats per minute) might be taken as non-critical. Thus, if a patient's pulse, previously above this range, was found in the latest sensor reading to have fallen below 120, this would lead the sensor to a determination of non-emergency.
S23: If no longer in emergency, the network device or sensor 11 sends an indicator of lifting the state of emergency to the coordinator 10. Of course, this "sending" need not be a direct transmission from the sensor to the coordinator. In a peer-to-peer topology, it may be that the sensor transmits only to its nearest neighbour, the message reaching the coordinator over a series of hops.
S24: The network device or sensor starts to listen for the ACK from coordinator 10.
S25: The coordinator sends back the ACK to the network device.
S26: Some actions will follow for lifting the emergency. For example, the sensor may reduce the frequency of its sensor readings, and/or communications with the coordinator, perhaps shutting down for more of the time to conserve power; the sensor may also suspend sending each sensor reading directly, perhaps storing them up for periodic transmission instead. In general, it will return to the non-emergency state which was assumed at the start of the flowchart of Figure 12. Meanwhile, the coordinator may re-allocate a GTS previously assigned to the sensor, for other purposes. The coordinator might also send a message externally to inform a higher authority (human or machine) that the emergency has passed.

In a second protocol or trigger type, it is assumed that the sensor itself cannot determine whether an emergency situation exists with respect to the sensed parameter. Often, this will be because the sensor has insufficient processing power to do so. Alternatively, it may be that the sensed parameter by itself is not enough to declare an emergency and other factors have to be taken into account. The coordinator 10 may be in a position to do this owing to its relatively higher processing power and ability to obtain information from other sources.

An example of such other factors would be time: it might be acceptable for the sensed life parameter to cross a threshold for a short time, or for an isolated reading, but perhaps a number of such readings within a certain time period would indicate an emergency state. As an example, the patient's pulse rate might momentarily rise to a critical level due to some external stimulus but fall quickly again; this would not indicate an emergency. Normally, the coordinator would have the storage and counting abilities to make such a judgement.

Another example of other factors would be sensor data or indications from sensors used to detect other life parameters of the same patient. One parameter in isolation might not allow conclusive determination of an emergency, but could do so when combined with information from other sensors.

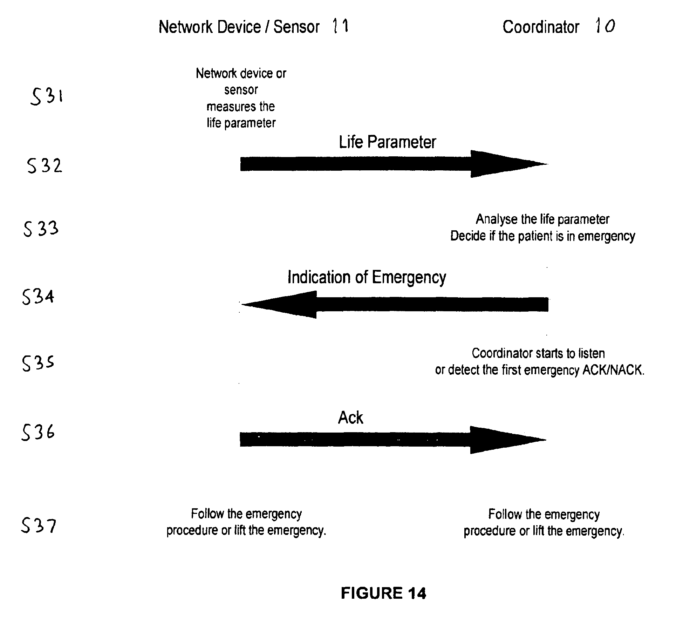

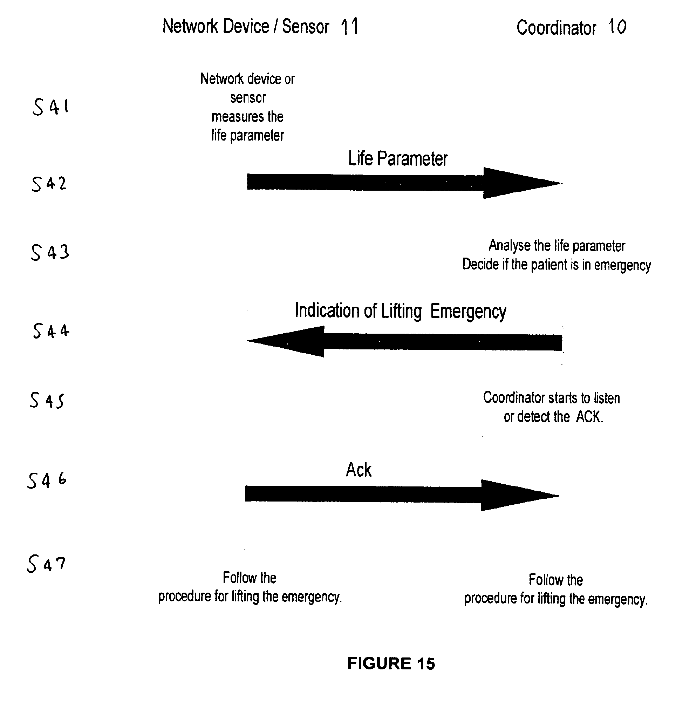

The process is similar to that just outlined, but with some differences as shown in Figures 14 and 15. Again, it is assumed in the first case (Fig. 14) that the initial state is non-emergency, and that the second case (Fig. 15) starts in the emergency condition.

Declaring Emergency, Trigger Type 2 (Figure 14):
S31: Network device or sensor 11 measures the life parameter, as before.
S32: The network device sends one or more sensed values of the life parameter to the coordinator. Incidentally, in any of the processes described, this could be by explicit signalling of the sensed value, and/or by sending a change in the value, or by sending some indication of the value such as a range in which it falls. Also, as mentioned, in the non-emergency state it might not be necessary to send every sensed value.
S33: Coordinator analyses the information to find out if the patient is "in emergency". This step may involve analysis of the sensed value in isolation or, as mentioned, could take other factors into consideration. Such factors could include the values sensed by other sensors on the same patient, or the time since the sensed parameter became critical.
S34: If an emergency exists, the coordinator 10 sends an emergency status confirmation to the network device. This action need not be confined to a confirmation sent to the specific network device in S31, but could be made to all devices in the network, or to a group of devices responsible for the same life parameter.
S35: Coordinator starts to listen for or detect an ACK from the sensor that it has received this confirmation.
S36: Network device sends back the ACK to the coordinator.
S37: Some emergency action will follow, as before. For example, the coordinator instructs the sensor to take sensor readings, and/or send an indication of such readings, more frequently than in the non-emergency state. In a simple configuration, receipt of the emergency status by the sensor at step S34 might be sufficient to initiate the necessary changes at the sensor, without the need for a separate instruction. However, more usually the emergency indication at S34 will need to be followed up in step S37 by a control message from the coordinator, perhaps informing the sensor of a new resource allocation for its transmissions.

Declaring lifting of Emergency, Trigger Type 2 (Figure 15):
S41: Network device or sensor measures the life parameter.
S42: Network device sends the life parameters to the coordinator.
S43: Coordinator analyses the information to find out if the patient is not in emergency anymore (i.e. whether the emergency state has ended and the non-emergency state has begun). This is essentially the inverse of the process in step S33, though any thresholds or time periods involved need not be the same. For example, a time period used to judge non-emergency might be set much longer than that for declaring an emergency, to ensure that the critical state has truly passed.
S44: If so, the coordinator sends an emergency status confirmation to the network device.
S45: Coordinator starts to listen or detect the ACK.
S46: Network device sends back the ACK to the coordinator.
S47: Some appropriate action will follow for lifting the emergency: for example the sensor may take the confirmation as a command to switch to a less active sleep/wake pattern to conserve energy.

The above is not the only possible way to declare lifting of an emergency. For example, in a situation where many emergencies have been declared in the same location such as hospital, it may be unhelpful to maintain the emergency state indefinitely, both in terms of congesting the network and overloading medical staff. Provision could be made, therefore, to lift the emergency after a predetermined time has elapsed. Such a provision might be applied selectively depending on the sensed parameter: for example a patient might be able to survive almost indefinitely with a very high blood pressure, enabling the emergency to be lifted if necessary, but not with a very low blood pressure.

The above two protocols only involve the sensor and the coordinator. However, an MBAN may be implemented in such a way that several coordinators report to some form of central monitor as already mentioned, which could be either automatic or under human supervision. For example, such a central monitor could be located at the desk of a ward sister who oversees several patients in a hospital. In this scenario, the central monitor can make the determination of whether or not an emergency exists, as follows.

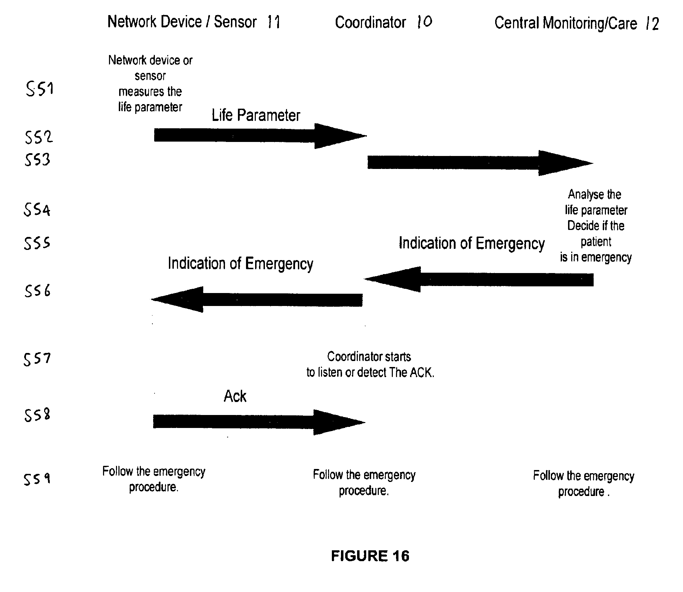

Declaring the State of Emergency, Trigger Type 3 (Figure 16):
S51: Assuming the network device or sensor 11 begins in the non-emergency state, it measures the life parameter.
S52: Network device sends the sensed life parameter data, in some form, to the coordinator 10.
S53: The coordinator 10 forwards the life parameter to the central monitoring unit 12 through some means. Note that the transmission means in this case need not be the MBAN itself; it could be a separate wireless communication network such as a mobile telephony network, or some form of wired connection such as a wired LAN in a hospital. Thus, the central monitor is not necessarily to be considered a part of the MBAN as such. Again, the data transmitted need not be a direct transmission of the sensed value, but might be a notification of reaching a certain threshold, or range. The way in which the coordinator indicates the sensed values to the central monitor need not be the same as that used by the sensor to transmit to the coordinator.
S54: The centralized monitoring system 12 analyses the information to find out if the patient is in emergency (has entered the emergency state). In this case, the determination may involve factors extraneous to the patient, such as the situation of any other patients being monitored (who may be in an even worse state and thus more deserving of attention). The final decision could be taken with human intervention, or could be automatic.
S55: If an emergency state is determined, the centralized monitoring system 12 sends an emergency status confirmation to the coordinator, normally by the same transmission route as in step S53.
S56: In response to the confirmation in S55, the coordinator also sends an emergency status confirmation to the network device.
S57: Coordinator starts to listen or detect the ACK.
S58: Network device sends back the ACK to the coordinator.
S59: Some emergency actions will follow (which need not wait for completion of steps S56 and S57). For example, an alarm could sound at the workstation of a medical staff member, to urge them to attend the patient. The central monitor could inform the emergency condition to, and/or initiate actions in, other network devices including further coordinators if present.

Likewise the central monitor may take the decision to lift the emergency, based on the sensed information as relayed or filtered through the coordinator as well as any other factors of which it is aware.

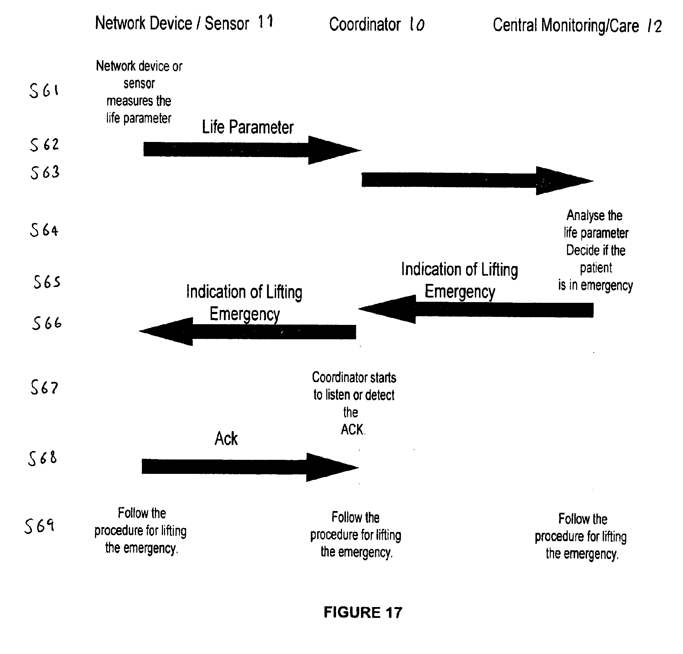

Declaring Lifting the State of Emergency, Trigger Type 3 (Figure 17):
S61: Starting in the emergency state, the network device or sensor 11 measures the life parameter.
S62: Network device send the life parameters to the coordinator 10.
S63: The coordinator forwards the life parameter to the central monitoring unit through some network, as in S53.
S64: The centralized monitoring system 12 analyses the information to find out if the patient is not in emergency, i.e. whether an existing emergency state has ended.
S65: If so, the centralized monitoring system sends an emergency status confirmation to the coordinator.
S66: In response, coordinator also sends an emergency status confirmation to the network device.
S67: Coordinator starts to listen or detect the ACK.
S68: Network device sends back the ACK to the coordinator.
S69: Some actions will follow; the network device may change its transmission and/or sleep/wake pattern as before, and for example the coordinator may confirm back to the central monitor 12 that the network device has been returned to the non-emergency condition.

Note that the order of steps need not follow strictly that shown in the flowcharts. For example, in Figure 12, the coordinator could combine sending the ACK with allocating a GTS (or increased GTS) and notifying the sensor of this fact. Likewise, either the sensor 11 or coordinator 10 could start to take emergency (or lifting of emergency) procedures as soon as it is aware of the change in emergency status, without waiting for an ACK from the other side.

It is not necessarily the case that "emergency" and "non emergency " are the only two possible conditions. For example, a third condition such as "abnormal" could be introduced to signify that the patient (or more correctly, the value of a certain sensed life parameter) is giving cause for concern though not yet in an emergency condition. This could either be declared explicitly by extending the above-described procedures, or defined implicitly by not returning to the non-emergency state right away, but rather waiting until the sensed value has returned to a normal reading. In other words, any threshold value(s) used to determine the emergency and non-emergency state respectively need not be the same. Alternatively, the degree of emergency could be raised or lowered in a series of steps depending on the seriousness of the situation, as explained in more detail below.

The flowcharts of Figures 12 to 17 consider a single sensor for the sake of simplicity. However, depending on the life parameter concerned, multiple sensors might be provided on or implanted in the same patient to monitor the same life parameter; for example, temperature. In such a case, of course, steps described with respect to the single sensor would in fact involve all of such multiple sensors.

Likewise, the procedures explained above might involve more than one coordinator, for example in a peer-to-peer setting where several clusters each with their own coordinator report to a single central monitoring unit. In this case, messages from a given coordinator to the central monitor need not always be transmitted directly but could be relayed by one or more other coordinators.

Although the above description has referred only to sensors and coordinators in a wireless sensor network, with the possible inclusion of a central monitor, it is possible for a MBAN to include other devices than these kinds. Potentially, some means of intervening in the patient's care, such as a drug dispensing mechanism, could be arranged in the network under wireless control of the coordinator and/or any central monitor. Thus, the "emergency procedure" need not be confined to control of sensors and their communications, but could extend for example to delivering a drug to the patient to stabilise a life parameter (heart rate, for instance) in an emergency state.

The above description has concerned techniques for determining a medical emergency or non-emergency of one or more patients, since this is seen as an important application of the present invention. However, it is not the only possible application. Sensors could be used to monitor a living body in non-medical situations. For example, any person at risk (examples: old or frail people, or children; people in dangerous environments, etc.) could be monitored using the same techniques as described above. In this case, the emergency condition would represent some form of physical threat such as an accident. Sensors for such life parameters such as pulse, temperature, acceleration etc. would be of particular use in this situation.

There are many possibilities for applying the present invention beyond the BAN of a human or other living body. One possibility is a WSN capable of detecting industrial emergencies such as many potential scenarios in a mission critical industrial environment (for example, power stations). This can apply to multiple control points in a factory environment. For example we may consider temperature sensors in a factory's heating facility or pressure thresholds for food product lines. The immediate ACK protocol may applied to emergencies in these systems just as for medical emergencies. Thus, the term "entity" in the claims is to be interpreted as covering any such industrial environment in addition to living beings.

Some description will now be given of how the above protocols may be accommodated within a communications standard, like IEEE 802.15.6 currently under development, drawn from IEEE 802.15.4.

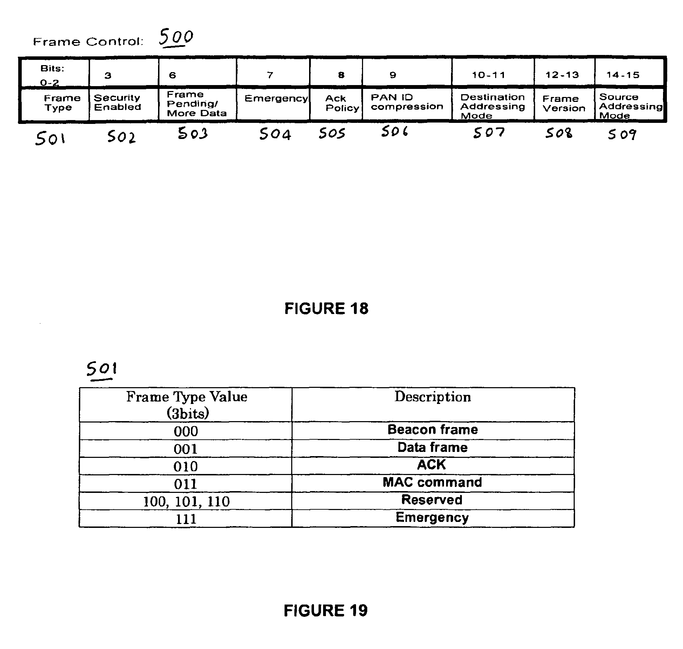

Figures 18 and 19 illustrates a first possible modification to the IEEE 802.15.4 frame format in one embodiment of the present invention, to accommodate the emergency situation through the addition of a new bit labelled "emergency" and make it suitable for IEEE 802.15.6. In this first possible modification, allowance is made for a novel emergency frame type but without making any other changes to the frame types in IEEE 802.15.4.

As already outlined, IEEE 802.15.4 provides various frame types including beacon frame 41, data frame 42, acknowledgement frame 43 and MAC Command frame 44. In IEEE 802.15.6, one way to implement the above-described procedures is to introduce a further frame type, the emergency frame, to the defined MAC frame types.

Figure 18 shows the structure of a Frame Control Field 500, corresponding to the Frame Control Field 50 of Figure 10A already proposed for IEEE 802.15.4. As will be seen by comparing Figure 18 with Figure 10A, bits 0-2 denote the frame type 501 as in IEEE 802.15.4, but the possible frame type values are changed as shown in Figure 19. Of the previously reserved values 100-111 (see Fig.10B), bit value "111" is now used to denote the novel emergency frame type. Values 100, 101 and 110 remain as reserved values for future use.

In the remaining subfields of the frame control field 500, basically the same components are present as in the frame control field 50 of Figure 10, except that bit no. 7 is newly used as a flag for the emergency state (for example: "1" = emergency and "0" = no emergency). Bit 8 is now used to represent an Ack policy (corresponding to the Ack request subfield of Fig.10A). The subfields for security enabled bit 502, Frame Pending bit 503, PAN ID compression 506, destination addressing mode 507, frame version 508 and source addressing mode 509 have the same functions as their counterparts in IEEE 802.15.4 frame control field 50.

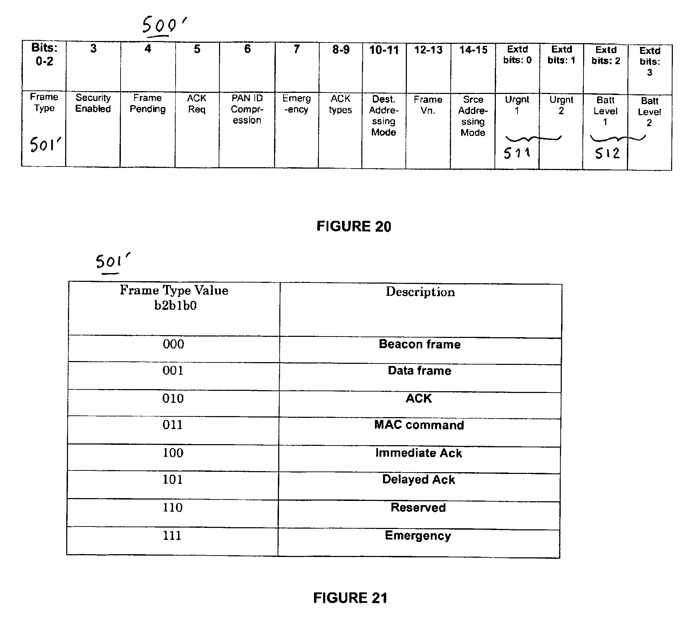

Figure 20 and 21 illustrate a second possible modification to the IEEE 802.15.4 frame format in another embodiment of the present invention, to accommodate not only the emergency frame type but other novel features including a more flexible ACK provision including a so-called immediate ACK used for example in emergency, an indication of the state of a battery of a network device, and an indication of "urgency".

The format of the frame control field 500' of Figure 20 differs from that 500 of Figure 18 mainly in that the single bit 505 for Ack policy is replaced by two bits for defining different ACK types, and in that indications of battery state (i.e. remaining charge or voltage level) and "urgency" are represented by new subfields 511 and 512 requiring additional bits (labelled "Extd bits" 0-3 in the Figure). As can be seen, two bits each are allocated to each of "Urgnt" and "Batt Level" allowing up to four levels to be defined for each. The meaning and use of these new subfields is outside the scope of the present invention, but it is noted here that they may be used in conjunction with the emergency status in the present invention to provide more flexible signalling between the devices of a BAN.

The IEEE 802.15.4 modified frame type values in this case are as shown in Figure 21, which should be compared with Figures 10A/10B and 19. Compared with the embodiment of Figure 19, the difference is that the previously-reserved values 100 and 101 are now used to denote two types of ACK, i.e. immediate ACK and delayed ACK, the immediate ACK being used e.g. by devices in emergency to acknowledge each individual frame of received data for a more reliable communication. The immediate ACK is the subject of a co-pending application by the same applicant.

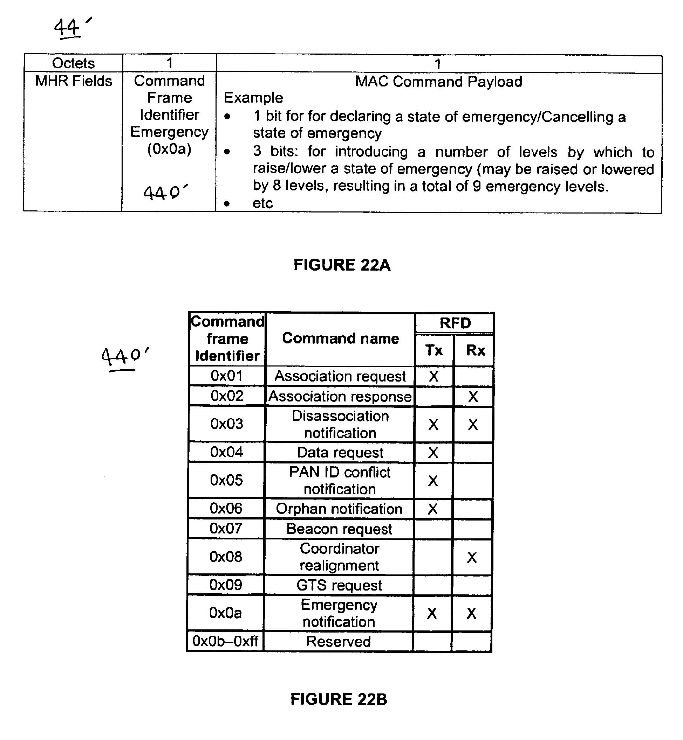

As a further technique for integrating the novel features of the present invention into frame structures already proposed, the command frame identifier of a MAC command frame (refer back to Figures 11A and 11B) may be used. Figures 22A and 22B show the modification required to a MAC command frame 44' including the addition of new command type "Emergency notification". Figure 22A shows part of the MAC command frame format 44' and Figure 22B shows the table of possible values for the command frame identifier 440', the new type "Emergency notification" occupying value 0x0a which was previously unused (see Figure 22B). In addition to defining the new command type, the payload following the command frame identifier 440' is used to give some information (context) for the command. In the case of MAC command frame 44' shown in Figure 22A, an example of a payload would be one bit for declaring a state of emergency or for cancelling (lifting) the emergency state: e.g. "1" = emergency, "0" = no emergency, followed by three bits for introducing a number of levels by which to raise/lower a state of emergency (allowing 8 levels of emergency for a total of 9 levels including the non-emergency state). Additional bits could also be provided as payload, for example to indicate a duration for the emergency declaration either in relative time (ms) or absolute time (ms since the start of the present epoch).

To summarise, an embodiment of the present invention may provide the following features and advantages:
* Ties the medical emergency situation to a new Emergency Acknowledge signalling for IEEE 802.15.6.
* Defines a new frame type "emergency".
* Introduces the possibility of a trigger type one for medical emergency where sensor or network device decides if there is an emergency.
* Provides an emergency trigger protocol associated with the trigger type one.
* Introduces the possibility of trigger type two for medical emergency where coordinator assesses the situation and decides if there is an emergency.
* Provides an emergency trigger protocol associated with the trigger type two.
* Introduces the possibility of trigger type three for medical emergency where coordinator forwards the life parameter to a central care system to assess the situation and decide if there is an emergency.
* Provides an emergency trigger protocol associated with the trigger type three.
* Defines a new control frame structure for IEEE 802.15.6.
* Defines a new bit in a control frame indicating an emergency situation for IEEE 802.15.6.

Embodiments of the present invention may have a vital role to play in facilitating emergency management by use of MBANs. The following scenarios may be noted:
(i) Hundreds of millions of patients worldwide with cardiac and heart problems can be monitored in hospital or at home by employing wireless sensors forming an MBAN on their bodies. The MBAN can provide extra mobility for such patients. However, for this group of patients under situations such as abnormal heart functioning or more severe cases such as heart attack, it is vital to secure a reliable communication channel to make sure that no emergency or alarm signal will be missed. The present invention provides a secure emergency trigger mechanism to make all the entities involved aware about an emergency by sending an "Emergency Acknowledge".
(ii) Hundreds of millions of people worldwide suffer from diabetes. Implantable or non-invasive methods for glucose measurement have been considered recently. An MBAN can be used to monitor a patient's glucose level information on a 24-hour basis. There are situations where the patient's glucose level is off the chart and emergency geolocation and other necessary urgent medical procedures for the patients are required.
(iii) MBANs may be used to gather sensed data while monitoring a patient in intensive care where the loss of data could be life threatening.
(iv) Improves the labour costs and efficiency of emergency response in a medical system.
(v) Improves the emergency awareness in a medical MBAN system.
(vi) Reduces the labour costs by automating the emergency response process.
(vii) Although primarily envisaged for low data-rate applications, MBANs could have application to transfer of streaming video/audio data where loss of individual packet is crucial and affects the quality. Erroneous data may have a negative impact on the diagnosis of illness in emergency cases.
(viii) For medical diagnosis, MMR or X-ray images need to be very clear in order for the doctor to diagnose properly the patient. Again, therefore, reliable data transfer is essential.

In summary, the present invention can provide a method of declaring an emergency condition in a wireless sensor network, the wireless sensor network comprising a plurality of devices (10, 11, 12) including sensors (11) arranged for monitoring at least one entity, the method comprising steps of: sensing a value of parameter related to the entity by one of the sensors (11); wirelessly transmitting information by the sensor to another device (10) in the network; determining existence or non-existence of an emergency condition affecting the entity by using the sensed value or the transmitted information; and declaring existence or non-existence of the emergency condition to at least one other device (e.g. 10) in the network. The determining step may be carried out in any of the sensor (11), a coordinator (10) of the wireless sensor network, or a central monitor (12) in communication with the coordinator. The declaring step may be performed by setting a frame control field of a frame-based wireless sensor network to a value predefined as denoting an emergency frame. An acknowledgement frame may be used by the recipient to acknowledge receipt of the emergency declaration, after which an emergency procedure is followed. The emergency procedure can involve, for example, increasing the bandwidth allocated to the sensor so as to ensure reliability of subsequent information from the sensor. The method may be applied, for example, to monitoring of patients in a hospital using MBANs operating in accordance with IEEE 802.15.6.

The present invention may take the form of a novel sensor, coordinator, or hardware modules for the same, and can be implemented by replacing or modifying software executed by processors of the sensor(s) and/or the coordinator.

Thus, embodiments of the present invention may be implemented in hardware, or as software modules running on one or more processors, or on a combination thereof. The invention may also be embodied as one or more device or apparatus programs (e.g. computer programs and computer program products) for carrying out part or all of any of the techniques described herein. Such programs embodying the present invention may be stored on computer-readable media, or could, for example, be in the form of one or more signals. Such signals may be data signals downloadable from an Internet website, or provided on a carrier signal, or in any other form.

Although the above description has referred to IEEE 802.15.4 and IEEE 802.15.6 by way of example, the invention may be applied to any type of frame-based wireless sensor network or MBAN whether or not operating in accordance with IEEE 802.15.6, as well as to other types of BAN which even if not medical body area networks nevertheless have a requirement for improved reliability of communication in emergency situations.

## Claims

1. A wireless sensor network of devices including one or more sensors (11) for monitoring an entity, including:
a said sensor arranged to detect a value of a parameter related to the entity and to wirelessly transmit information to another device (10, 11, 12) in the network;
a coordinator (10) arranged to receive said wirelessly transmitted information; and
determining means responsive to said detected value or said transmitted information to determine existence of an emergency condition of the entity, the determining means arranged to transmit a declaration of the emergency condition to at least one other device (10, 11, 12) in the wireless sensor network; **characterised in that**
information is transmitted in the network via Medium Access Control frames, and the determining means is arranged to make the declaration of the emergency condition by setting a Medium Access Control frame identifier to an emergency notification frame type.

2. The wireless sensor network according to claim 1 wherein said determining means is located in the sensor (11).

3. The wireless sensor network according to claim 1 wherein said determining means is located in the coordinator (10).

4. The wireless sensor network according to claim 1 wherein there is provided a central monitor (12) in communication with said coordinator (10), and said determining means is provided by the central monitor.

5. The wireless sensor network according to any preceding claim wherein said determining means makes said determination by comparing said detected value or said transmitted information with at least one threshold value.

6. The wireless sensor network according to claim 5 wherein said determining means is further operable to determine non-existence of the emergency condition by comparing said detected value or said transmitted information with at least one threshold value.

7. The wireless sensor network according to claim 1 wherein said other device (10, 11, 12) is responsive to said declaration to send an acknowledgement to the determining means.

8. A sensor (11) for use in the wireless sensor network of claim 2 and including said determining means.

9. A coordinator (10) for use in the wireless sensor network of claim 3 and including said determining means.

10. A method of declaring an emergency condition in a wireless sensor network, the wireless sensor network comprising a plurality of devices including sensors (11) arranged for monitoring at least one entity, the method comprising steps of:
sensing a value of parameter related to the entity by one of the sensors (11);
wirelessly transmitting information by said sensor to another device (10, 11, 12) in the network;
determining existence or non-existence of an emergency condition affecting said entity by using said sensed value or said transmitted information; and
declaring existence or non-existence of the emergency condition to at least one other device (10, 11, 12) in the network; **characterised in that**
information is transmitted in the network via Medium Acces Control frames, and said declaring step is performed by setting a Medium Acces Control frame identifier to an emergency notification frame type.

## Patentansprüche

1. Drahtloses Sensornetz von Vorrichtungen mit einem einen oder mehreren Sensoren (11) zum Überwachen einer Person hat, umfassend:
einen derartigen Sensor, der angeordnet ist, um einen Wert eines Parameters bezüglich der Person zu detektieren und Informationen drahtlos an eine andere Vorrichtung (10, 11, 12) in dem Netz zu senden;
einen Koordinator (10), der angeordnet ist, um die drahtlos gesendeten Informationen zu empfangen; und
ein Bestimmungsmittel, das auf den detektierten Wert oder die gesendeten Informationen reagiert, um das Vorliegen eines Notfalls der Person zu bestimmen, wobei das Bestimmungsmittel angeordnet ist, um eine Meldung des Notfalls an wenigstens eine andere Vorrichtung (10, 11, 12) in dem drahtlosen Sensornetz zu senden; **dadurch gekennzeichnet, dass**
Informationen in dem Netz über Medium-Access-Control-Rahmen gesendet werden und das Bestimmungsmittel angeordnet ist, um die Meldung des Notfalls vorzunehmen, indem ein Medium-Access-Control-Rahmen-Identifier auf einen Notfallnachricht-Rahmentyp gesetzt wird.

2. Drahtloses Sensornetz nach Anspruch 1, bei dem das Bestimmungsmittel in dem Sensor (11) positioniert ist.

3. Drahtloses Sensornetz nach Anspruch 1, bei dem das Bestimmungsmittel in dem Koordinator (10) positioniert ist.

4. Drahtloses Sensornetz nach Anspruch 1, bei dem ein Zentralmonitor (12) vorgesehen ist, der mit dem Koordinator (10) in Verbindung steht, und das Bestimmungsmittel durch den Zentralmonitor vorgesehen wird.

5. Drahtloses Sensornetz nach einem vorhergehenden Anspruch, bei dem das Bestimmungsmittel die Bestimmung vornimmt, indem der detektierte Wert oder die gesendeten Informationen mit wenigstens einem Schwellenwert verglichen werden.

6. Drahtloses Sensornetz nach Anspruch 5, bei dem das Bestimmungsmittel ferner betriebsfähig ist, um das Nichtvorliegen des Notfalls zu bestimmen, indem der detektierte Wert oder die gesendeten Informationen mit wenigstens einem Schwellenwert verglichen werden.

7. Drahtloses Sensornetz nach Anspruch 1, bei dem die andere Vorrichtung (10, 11, 12) auf die Meldung reagiert, um eine Bestätigung an das Bestimmungsmittel zu schicken.

8. Sensor (11) zur Verwendung in dem drahtlosen Sensornetz nach Anspruch 2, der das Bestimmungsmittel enthält.

9. Koordinator (10) zur Verwendung in dem drahtlosen Sensornetz nach Anspruch 3, der das Bestimmungsmittel enthält.

10. Verfahren zum Melden eines Notfalls in einem drahtlosen Sensornetz, welches drahtlose Sensornetz eine Vielzahl von Vorrichtungen umfasst, die Sensoren (11) enthalten, die zum Überwachen wenigstens einer Person angeordnet sind, wobei das Verfahren die Schritte umfasst:
Fühlen eines Wertes eines Parameters bezüglich der Person durch einen der Sensoren (11);
drahtloses Senden von Informationen durch den genannten Sensor an eine andere Vorrichtung (10, 11, 12) in dem Netz;
Bestimmen des Vorliegens oder Nichtvorliegens eines Notfalls, der die Person betrifft, unter Verwendung des gefühlten Wertes oder der gesendeten Informationen; und
Melden des Vorliegens oder Nichtvorliegens des Notfalls an wenigstens eine andere Vorrichtung (10, 11, 12) in dem Netz; **dadurch gekennzeichnet, dass**
Informationen in dem Netz über Medium-Access-Control-Rahmen gesendet werden und der Meldungsschritt ausgeführt wird, indem ein Medium-Access-Control-Rahmen-Identifier auf einen Notfallnachricht-Rahmentyp gesetzt wird.

## Revendications

1. Réseau de capteurs sans fil de dispositifs comprenant un ou plusieurs capteurs (11) pour surveiller une entité, comprenant :
ledit capteur étant agencé pour détecter une valeur d'un paramètre ayant trait à l'entité et pour transmettre sans fil une information à un autre dispositif (10, 11, 12) dans le réseau ;
un coordinateur (10) agencé pour recevoir ladite information transmise sans fil ; et
un moyen de détermination sensible à ladite valeur détectée à ladite information transmise pour déterminer l'existence d'une condition d'urgence de l'entité, le moyen de détermination étant agencé pour transmettre une déclaration de la condition d'urgence à au moins un autre dispositif (10, 11, 12) dans le réseau de capteurs sans fil ; **caractérisé en ce que**
l'information est transmise dans le réseau par l'intermédiaire de trames de contrôle d'accès au support, et le moyen de détermination est agencé pour effectuer la déclaration de la condition d'urgence en fixant un identifiant de trame de contrôle d'accès au support à un type de trame de notification d'urgence.

2. Réseau de capteurs sans fil selon la revendication 1, dans lequel ledit moyen de détermination est situé dans le capteur (11).

3. Réseau de capteurs sans fil selon la revendication 1, dans lequel ledit moyen de détermination est situé dans le coordinateur (10).

4. Réseau de capteurs sans fil selon la revendication 1, dans lequel est prévu un moniteur central (12) en communication avec ledit coordinateur (10), et ledit moyen de détermination est délivré par le moniteur central.

5. Réseau de capteurs sans fil selon l'une quelconque des revendications précédentes, dans lequel ledit moyen de détermination effectue ladite détermination en comparant ladite valeur détectée ou ladite information transmise à au moins une valeur de seuil.

6. Réseau de capteurs sans fil selon la revendication 5, dans lequel ledit moyen de détermination permet en outre de déterminer la non-existence de la condition d'urgence en comparant ladite valeur détectée ou ladite information transmise à au moins une valeur de seuil.

7. Réseau de capteurs sans fil selon la revendication 1, dans lequel ledit autre dispositif (10, 11, 12) est sensible à ladite déclaration pour envoyer un accusé de réception au moyen de détermination.

8. Capteur (11) utilisé dans le réseau de capteurs sans fil selon la revendication 2, et comprenant ledit moyen de détermination.

9. Coordinateur (10) utilisé dans le réseau de capteurs sans fil selon la revendication 3 et comprenant ledit moyen de détermination.

10. Procédé pour déclarer une condition d'urgence dans un réseau de capteurs sans fil, le réseau de capteurs sans fil comportant une pluralité de dispositifs comprenant des capteurs (11) agencés pour surveiller au moins une entité, le procédé comportant les étapes consistant à :
détecter une valeur d'un paramètre ayant trait à l'entité par l'un des capteurs (11) ;
transmettre sans fil une information par ledit capteur à un autre dispositif (10, 11, 12) dans le réseau ;
déterminer l'existence ou la non-existence d'une condition d'urgence affectant ladite entité en utilisant ladite valeur détectée ou ladite information transmise ; et
déclarer l'existence ou la non-existence de la condition d'urgence à au moins un autre dispositif (10, 11, 12) dans le réseau ; **caractérisé en ce que**
une information est transmise dans le réseau par l'intermédiaire de trames de contrôle d'accès au support, et ladite étape de déclaration est effectuée en fixant un identifiant de trame de contrôle d'accès au support à un type de trame de notification d'urgence.
